# EUROPÄISCHE PATENTSCHRIFT

(11) **EP 2 559 379 B1**
(45) Veröffentlichungstag und Bekanntmachung des Hinweises auf die Patenterteilung: **13.09.2017**
(21) Anmeldenummer: 12174864.4
(22) Anmeldetag: 04.07.2012
(51) Int. Cl.: A61B 5/107, G01B 3/16

(54) **Messzirkel zum Vermessen des menschlichen Körpers**
Compass for measuring the human body
Compas de mesure destiné au corps humain

(30) Priorität: 16.08.2011 AT 45311 U
(43) Veröffentlichungstag der Anmeldung: 20.02.2013
(73) Patentinhaber: Wenatex Forschung-Entwicklung-Produktion GmbH, 5020 Salzburg (AT)
(72) Erfinder: Wernicke, Hans-Gerd, 83395 Freilassing (DE)
(74) Vertreter: Müllner, Martin

(56) Entgegenhaltungen:
- WO-A1-2008/111784
- DE-A1-102006 004 514
- DE-C1- 3 990 155
- GB-A- 2 408 020
- US-A- 3 955 285
- US-A1- 2001 037 581

## Beschreibung

### Technisches Gebiet

Die Erfindung betrifft einen Messzirkel, insbesondere zum Vermessen des menschlichen Körpers, umfassend zwei mit einem Gelenk verbundene Schenkel, welche an ihren freien Enden jeweils eine Messspitze aufweisen, sowie eine Messelektronik, welche in einem Schenkel untergebracht ist, und welche die jeweilige Stellung der beiden Schenkel zueinander erfasst.

### Stand der Technik

Ein derartiger Messzirkel ist beispielsweise aus der WO 00/06968 bekannt. Solche Messzirkel können unter anderem eingesetzt werden, um den menschlichen Körper abzumessen und so individuelle Unterschiede quantitativ zu erfassen. Die erhaltenen Messwerte können dann beispielsweise verwertet werden, um Produkte, wie Matratzen, Lattenroste, Sitzmöbel oder dergleichen, optimal auf die jeweils vermessene Person einzustellen. Der Messzirkel kann verschiedene Maße wie Schulterbreite, Hüftbreite oder Länge der Wirbelsäule erfassen. Um beispielsweise einen Lattenrost optimal einzustellen, ist zusätzlich die jeweilige Lordose der zu vermessenden Person zu bestimmen. Dies ist vorläufig nur mit zusätzlichen aufwändigen Gerätschaften möglich. Weitere dem Stand der Technik entsprechende Messzirkel sind zum Beispiel in DE3990155C1 und WO2008/111784A1 offenbart.

### Kurzbeschreibung der Erfindung

Ziel der Erfindung ist es, einen Messzirkel zu schaffen, welcher es erlaubt zusätzlich zu den bisherigen Funktionen auch die Lordose einer Person zu bestimmen, wobei das Gerät kompakt und einfach im Aufbau sein soll, und die erfassten Werte in elektronischer Form für die Weiterverarbeitung bereitgestellt werden sollen.

Diese Aufgabe wird durch den Messzirkel gemäß Anspruch 1 gelöst. An einem Schenkel ist ein weiterer Messstab zu diesem Schenkel verstellbar angeordnet, wobei die Messelektronik den Normalabstand zwischen der Längsachse des Schenkels und der Spitze des Messstabs erfasst. Für die Vermessung der Lordose wird der Messzirkel auf 180° aufgeklappt und in Längsrichtung an die Wirbelsäule der zu vermessenden Person gehalten. Der verstellbare Messstab wird dann mit seiner Spitze an den tiefsten Punkt der jeweiligen Lordose bewegt und die Messelektronik zeichnet an dieser Position den entsprechenden Wert auf.

Ein Merkmal ist es, dass die Messspitzen der beiden Schenkel abgerundet sind und zueinander gerichtet angeordnet sind, wobei sich die Messspitzen im zusammengeklappten Zustand des Messzirkels gegebenenfalls überlappen. Durch die abgerundeten Spitzen ist eine sehr sanfte Messung möglich, wobei zusätzlich die zu vermessende Person nicht von dem Messgerät abgeschreckt wird. Die überlappenden Spitzen erlauben es, den Messzirkel vollständig zusammenzuklappen, wodurch ein kompakter Transport möglich ist.

Ein weiteres Merkmal ist es, dass die Messelektronik über eine Schnittstelle zur drahtlosen Datenübertragung an einen Computer verfügt. Die gemessenen Daten können so leicht mittels unterschiedlicher Software direkt weiterverarbeitet werden.

Gemäß einem weiteren Merkmal ist es vorgesehen, dass am Messzirkel eine Anzeige sowie eine Eingabeeinrichtung angeordnet sind. Die unterschiedlichen zu erfassenden Messwerte können somit direkt am Zirkel ausgewählt und angezeigt werden.

Ferner ist es vorgesehen, dass die Schenkel in einer Stellung von 180° zueinander arretierbar sind. Dies erleichtert die Handhabung des Geräts bei der Vermessung der Lordose.

Gemäß einer möglichen Ausführungsform des Messzirkels ist es vorgesehen, dass der Messstab in einer Führung im Schenkel normal zu diesem verschiebbar angeordnet ist.

Entsprechend einer alternativen Ausführungsform des Messzirkels ist der Messstab über ein Gelenk mit dem Schenkel verbunden, und der Messstab ist im eingeklappten Zustand in einer Ausnehmung im Schenkel aufgenommen. Diese Ausführungsform erlaubt ebenfalls einen sehr kompakten Aufbau, was den einfachen Transport des Messzirkels erleichtert.

### Kurze Beschreibung der Zeichnungsfiguren

Die Erfindung wird nun anhand der beiliegenden Zeichnungen näher erläutert, wobei
Fig. 1 eine perspektivische Ansicht eines Messzirkels zeigt, und
Fig. 2 eine weitere perspektivische Ansicht des Messzirkels aus Fig. 1 in aufgeklappter Stellung zur Messung der Lordose zeigt.

### Beschreibung der Ausführungsarten

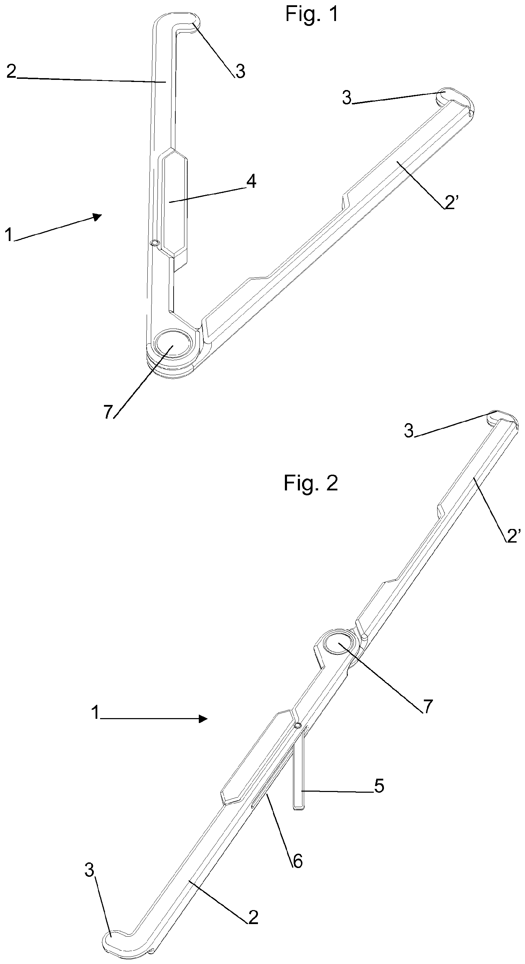

Der in Fig. 1 dargestellte Messzirkel 1 umfasst zwei mittels eines Gelenks 7 miteinander verbundene Schenkel 2. Am freien Ende der Schenkel 2 sind Messspitzen 3 vorgesehen, welche zueinander geneigt und abgerundet sind. Sie sind so an den Schenkeln 2 angeordnet, dass sie im zusammengeklappten Zustand überlappen, wodurch der Messzirkel 1 kompakt zusammenklappbar ist. In einem der Schenkel 2 ist ferner eine Messelektronik 4 untergebracht, welche zum Aufzeichnen der Messwerte dient. In einer Ausnehmung 6 eines Schenkels 2 ist ferner ein Messstab 5 angeordnet und über ein Gelenk mit dem Schenkel 2 verbunden. Dieser Messstab dient zur Vermessung der Lordose einer zu vermessenden Person. Die Messelektronik kann Messwerte aufgrund der Stellung der Schenkel 2 zueinander sowie aufgrund der Stellung des Messstabs 5 zum mit diesem verbundenen Schenkel 2 empfangen, verarbeiten und speichern sowie gegebenenfalls über eine Drahtlosschnittstelle an einen Computer übertragen. Beispielsweise bei der Vermessung einer Person für die individuelle Einstellung eines Lattenrosts kann der jeweilige Vertreter vor Ort den Messzirkels einsetzen, um sämtliche relevanten Werte, wie etwa Schulterbreite, Hüftbreite, Länge der Wirbelsäule und Lordose der zu vermessenden Person zu erfassen und somit den Lattenrost individuell anzupassen. Dabei benötigt er nur ein einziges Messgerät, welches aufgrund seiner kompakten Bauweise einfach zu transportieren ist und somit ideal im Außendienst beim Kunden vor Ort eingesetzt werden kann.

Die Fig. 2 zeigt den Messzirkel 1 in einer aufgeklappten Stellung, in welcher die Lordose vermessen werden kann. Die beiden Schenkel 2 stehen 180° zueinander und können gegebenenfalls in dieser Stellung arretiert werden. Der aufgeklappte Messzirkel wird senkrecht an die aufrecht stehende, zu vermessende Person entlang der Wirbelsäule angehalten. Durch Ausklappen des Messstabs 5 und Verschieben des Messzirkels in senkrechter Richtung kann somit mit der Spitze des Messstabs 5 der jeweils tiefste Punkt der Lordose der zu vermessenden Person erfasst werden. Die Messelektronik berechnet dabei aufgrund der Stellung des Messstabs 5 den Normalabstand zwischen Längsachse der Schenkel 2 zur Spitze des ausgeklappten Messstabs 5.

## Patentansprüche

1. Messzirkel (1) zum Vermessen des menschlichen Körpers, umfassend zwei mit einem Gelenk (7) verbundene Schenkel (2, 2'), welche beide an ihren freien Enden jeweils eine Messspitze (3) aufweisen, sowie eine Messelektronik (4), welche in einem der beiden Schenkel (2,2') untergebracht ist, und welche ausgebildet ist, die jeweilige Stellung der beiden Schenkel (2,2') zueinander zu erfassen, **dadurch gekennzeichnet, dass** an einem ersten Schenkel (2) ein weiterer Messstab (5) zur Vermessung der Lordose zu diesem Schenkel (2) verstellbar angeordnet ist, wobei die Spitze des Messstabs (5) sich in Messstellung auf der vom zweiten Schenkel (2') abgewandten Seite des ersten Schenkels (2) erstreckt, und wobei die Messelektronik (4) ausgebildet ist, den Normalabstand zwischen der Längsachse des ersten Schenkels (2) und der Spitze des Messstabs (5) zu erfassen.

2. Messzirkel nach Anspruch 1, **dadurch gekennzeichnet, dass** die Messspitzen (3) der beiden Schenkel (2,2') abgerundet sind und zueinander gerichtet angeordnet sind, wobei sich die Messspitzen (3) im zusammengeklappten Zustand des Messzirkels (1) gegebenenfalls überlappen.

3. Messzirkel nach Anspruch 1 oder 2, **dadurch gekennzeichnet, dass** die Messelektronik (4) über eine Schnittstelle zur drahtlosen Datenübertragung an einen Computer verfügt.

4. Messzirkel nach einem der Ansprüche 1 bis 3, **dadurch gekennzeichnet, dass** am Messzirkel (1) eine Anzeige sowie eine Eingabeeinrichtung angeordnet sind.

5. Messzirkel nach einem der Ansprüche 1 bis 4, **dadurch gekennzeichnet, dass** die Schenkel (2, 2') in einer Stellung von 180° zueinander arretierbar sind.

6. Messzirkel nach einem der Ansprüche 1 bis 5, **dadurch gekennzeichnet, dass** der Messstab (5) in einer Führung im ersten Schenkel (2) normal zu diesem verschiebbar angeordnet ist.

7. Messzirkel nach einem der Ansprüche 1 bis 5, **dadurch gekennzeichnet, dass** der Messstab (5) über ein Gelenk mit dem ersten Schenkel (2) verbunden ist, und dass der Messstab (5) im eingeklappten Zustand in einer Ausnehmung (6) im ersten Schenkel (2) aufgenommen ist.

## Claims

1. Measuring calliper (1) for measuring the human body, comprising two limbs (2, 2') that are connected to an articulated joint (7), said limbs both comprising on their free ends in each case a measuring tip (3), and also an electronic measuring system (4) that is housed in one of the two limbs (2, 2') and that is configured so as to ascertain the respective position of the two limbs (2, 2') with respect to one another, **characterised in that** a further measuring stick (5) is arranged on a first limb (2) in an adjustable manner with respect to said limb (2) so as to measure the lordosis, wherein the tip of the measuring stick (5) extends in the measuring position on the side of the first limb (2) that is remote from the second limb (2'), and wherein the electronic measuring system (4) is configured so as to ascertain the normal distance between the longitudinal axis of the first limb (2) and the tip of the measuring stick (5).

2. Measuring calliper according to claim 1, **characterised in that** the measuring tips (3) of the two limbs (2, 2') are rounded and are arranged oriented with respect to one another, wherein the measuring tips (3) overlap where appropriate when the measuring calliper (1) is in the folded-together state.

3. Measuring calliper according to claim 1 or 2, **characterised in that** the electronic measuring system (4) comprises an interface for wireless data transmission to a computer.

4. Measuring calliper according to any one of the claims 1 to 3, **characterised in that** a display and also an input device are arranged on the measuring calliper (1).

5. Measuring calliper according to any one of the claims 1 to 4, **characterised in that** the limbs (2, 2') can be locked in a position of 180° with respect to one another.

6. Measuring caliper according to any one of the claims 1 to 5, **characterised in that** the measuring stick (5) is arranged in a guiding arrangement in the first limb (2) in such a manner that said measuring stick can be displaced normally with respect to said limb.

7. Measuring caliper according to any one of the claims 1 to 5, **characterised in that** the measuring stick (5) is connected to the first limb (2) by way of an articulated joint, and that the measuring stick (5) is received in the folded state in a recess (6) in the first limb (2).

## Revendications

1. Compas (1) destiné à mesurer le corps humain, comprenant deux bras (2, 2') qui sont reliés par une articulation (7) et qui sont pourvus, chacun à son extrémité libre, d'une pointe de mesure (3) ainsi qu'une électronique de mesure (4) laquelle est logée dans l'un des deux bras (2, 2') et laquelle est réalisée de manière à détecter la position actuelle des deux bras (2, 2') l'un par rapport à l'autre, **caractérisé en ce que** le premier bras (2) est pourvu d'une tige de mesure (5) supplémentaire réglable par rapport à ce bras (2) permettant de mesurer la lordose , la pointe de la tige de mesure (5) s'étendant, en position de mesure, sur le côté du premier bras (2) qui se trouve à l'opposé du deuxième bras (2'), et l'électronique de mesure (4) étant réalisée de manière à détecter la distance normale entre l'axe longitudinal du premier bras (2) et la pointe de la tige de mesure (5).

2. Compas selon la revendication 1, **caractérisé en ce que** les pointes de mesure (3) des deux bras (2, 2') sont arrondies et disposées de manière à se faire face, les pointes de mesure (3) se chevauchant éventuellement lorsque le compas (1) est replié sur lui-même.

3. Compas selon les revendications 1 ou 2, **caractérisé en ce que** l'électronique de mesure (4) est pourvue d'une interface de transfert de données sans fil vers un ordinateur.

4. Compas selon l'une des revendications 1 à 3, **caractérisé en ce que** le compas (1) est pourvu d'un affichage ainsi que d'un dispositif de saisie.

5. Compas selon l'une des revendications 1 à 4, **caractérisé en ce que** les bras (2, 2') peuvent être immobilisés dans une position de 180° l'un par rapport à l'autre.

6. Compas selon l'une des revendications 1 à 5, **caractérisé en ce que** la tige de mesure (5) est disposée dans un guidage au sein du premier bras (2), de manière à pouvoir être déplacée selon la normale à ce dernier.

7. Compas selon l'une des revendications 1 à 5, **caractérisé en ce que** la tige de mesure (5) est reliée au premier bras (2) à travers une articulation et que la tige de mesure (5) est logée, en position repliée, dans un évidement (6) prévu dans le premier bras (2).
